# EUROPÄISCHE PATENTANMELDUNG

(11) **EP 0 816 320 A1**
(43) Veröffentlichungstag der Anmeldung: **07.01.1998**
(21) Anmeldenummer: 97109899.1
(22) Anmeldetag: 18.06.1997
(51) Int. Cl.: C07C 43/205, C07C 43/20, C07C 43/164, C07C 41/16, C07C 45/50

(54) **Neue einen 2,2'-Biarylrest enthaltende Bisether und ein Verfahren zu ihrer Herstellung**

(30) Priorität: 24.06.1996 DE 19625167
(71) Anmelder: HOECHST AKTIENGESELLSCHAFT, 65929 Frankfurt am Main (DE)
(72) Erfinder: Regnat, Dieter, Dr., 65817 Eppstein (DE); Kleiner, Hans-Jerg, Dr., 61476 Kronberg (DE); Bahrmann, Helmut, Dr., 46499 Hamminkeln (DE)

(57) **Zusammenfassung**

Die vorliegende Erfindung betrifft Verbindungen der allgemeinen Formel (I) 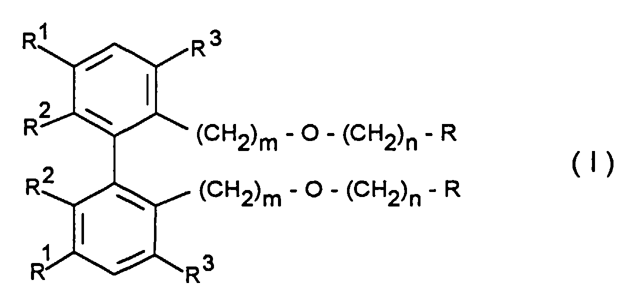 worin R¹, R² und R³ gleich oder verschieden sind und unabhängig voneinander für Wasserstoff, eine Alkyl- oder Alkoxygruppe mit 1 bis 4 Kohlenstoffatomen, eine Alkenylgruppe mit 2 bis 4 Kohlenstoffatomen stehen oder R¹ und R² unter Einschluß der jeweils mit ihren verbundenen Kohlenstoffatomen einen Ring mit 6 Kohlenstoffatomen bilden, m und n unabhängig voneinander 0 oder 1 und (m + n) gleich 1 oder 2 ist, und R für einen unsubstituierten oder durch eine Alkylgruppe mit 1 bis 4 Kohlenstoffatomen, eine Amino- oder Dialkylaminogruppe mit insgesamt 2 bis 8 Kohlenstoffatomen substituierten Phenyl- oder Naphthylrest steht sowie ein Verfahren zu ihrer Herstellung.

## Beschreibung

Aldehyde stellen aufgrund ihrer chemischen Eigenschaften eine wichtige Gruppe organischer Verbindungen dar. Sie lassen sich beispielsweise durch Aldolreaktion mit sich selbst oder einer anderen C-H aciden Verbindung (Methylenkomponente) in die entsprechenden Aldole oder nach Dehydratisierung des Aldols in die entsprechenden ungesättigten Kondensationsprodukte überführen. Ferner lassen sich Aldehyde zu den entsprechenden Carbonsäuren oxidieren oder zu den entsprechenden Alkoholen reduzieren. Durch Umsetzung von Aldehyden mit Ammoniak oder Aminen sind Imine oder Schiff'sche Basen zugänglich, die durch Umsetzung mit Wasserstoff die entsprechenden Amine ergeben.

Aldehyde erhält man in großtechnischem Maßstab durch die Hydroformylierung von olefinischen Verbindungen. Infolge der Reaktion der Kohlenstoff-Kohlenstoff-Doppelbindung mit Kohlenmonoxid und Wasserstoff bilden sich - wie die nachfolgende Reaktionsgleichung anhand eines endständigen Olefins schematisch belegt- Gemische von geradkettigen und verzweigten Aldehyden

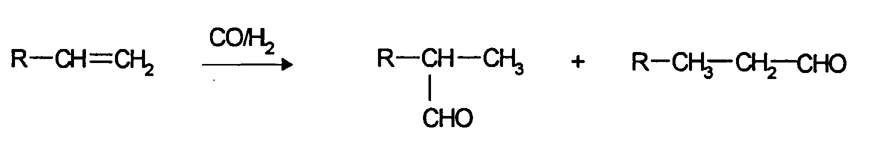

Die Gemische fallen in Abhängigkeit von den Reaktionsbedingungen in unterschiedlicher Zusammensetzung an. In vielen Fällen sind Gemische mit einem möglichst hohen Anteil geradkettiger Aldehyde und einem möglichst geringen Anteil verzweigter Aldehyde erwünscht. Neben den Reaktionsbedingungen wie Druck und Temperatur übt der verwendete Hydroformylierungskatalysator einen entscheidenden Einfluß auf den Ablauf der Reaktion und die Zusammensetzung des Reaktionsgemisches aus.

Bei der Hydroformylierung von Olefinen haben sich als Hydroformylierungskatalysator besonders Rhodium-Katalysatoren, die Phosphor enthaltende Liganden enthalten, bewährt. Geeignete Phosphor enthaltende Liganden sind Phosphane oder Phosphite. In der DE 17 93 069 ist ein derartiges Hydroformylierungsverfahren beschrieben.

Von Nachteil ist jedoch, daß die Phosphite und insbesondere die Phosphane gegenüber Sauerstoff und Schwefel nicht stabil sind und selbst durch sehr geringe Mengen Sauerstoff und/oder Schwefel oxidiert werden. Dabei entstehen Phosphate, Thiophosphate, Phosphanoxide und/oder Phosphansulfide.

Der Sauerstoff gelangt hauptsächlich über das als Ausgangsmaterial eingesetzte Olefin in die Reaktion, während der Schwefel in Formen schwefelhaltiger Verbindungen, beispielsweise als H₂S, über das Synthesegas der Umsetzung zugeführt wird.

Sauerstoff und/oder Schwefel wirken bereits in kleinsten Mengen schädlich, da üblicherweise der Katalysator nach erfolgter Hydroformylierung vom Reaktionsprodukt, beispielsweise durch Destillation, abgetrennt und in die Stufe der Hydroformylierung wieder eingesetzt wird. Dort gelangt er wiederum mit dem aus dem Einsatzolefin entstammenden Sauerstoff und dem mit dem Synthesegas zugeführten Schwefel bzw. den schwefelhaltigen Verbindungen in Kontakt. Als Folge hiervon werden weitere Anteile Phosphit oder Phosphan mit Sauerstoff und/oder Schwefel umgesetzt.

Die resultierenden Phosphate, Thiophosphate, Phosphanoxide und Phosphansulfide fungieren nicht mehr als komplexierender Ligand und sind somit nicht mehr katalytisch aktiv. Schwefelhaltige Verbindungen beinträchtigen zudem häufig katalytische Prozesse und wirken als Katalysatorgifte.

Die gebildeten Phosphate, Thiophosphate, Phosphanoxide und Phosphansulfide sind in der Hydroformylierung unerwünscht und müssen daher abgetrennt werden. Sowohl die Abtrennung als auch die Aufarbeitung des noch aktiven Katalysators erweist sich als schwierig und erfordert einen hohen technischen Aufwand.

Die Phosphite sind zwar etwas weniger empfindlich gegenüber Sauerstoff und/oder Schwefel, als die Phosphane. Sie sind jedoch empfindlich gegenüber Wasser und hydrolysieren auch unter dem Einfluß geringer bis sehr geringer Mengen Feuchtigkeit. Kleine Mengen Wasser gelangen über das eingesetzte Olefin und das Synthesegas in die Reaktion. Durch die Rückführung des die Phosphite enthaltenden Katalysators kommen sie immer wieder mit dem aus dem Olefin und Synthesegas entstammenden Wasser zusammen, was zur Folge hat, daß die Hydrolyse fortschreitet und immer mehr Phosphit hydrolytisch gespalten wird. Die Hydrolyseprodukte der Phosphite wirken nicht mehr komplexierend und sind auch nicht mehr katalytisch wirksam.

Im Hinblick auf die mit dem Einsatz von Phosphiten und Phosphanen beschriebenen Nachteile besteht ein Bedarf, Stoffe herzustellen, die unempfindlich gegenüber Sauerstoff und/oder Schwefel sind und auch nicht unter den Bedingungen der Hydroformylierung hydrolysieren. Sie sollen darüberhinaus komplexierende Eigenschaften besitzen, die Aktivität des Hydroformylierungskatalysators oder des Hydroformylierungskatalysatorsystems nicht herabsetzen und sicherstellen, daß es auch bei längerem Gebrauch nicht zu einer Desaktivierung des Hydroformylierungskatalysators oder des Hydroformylierungskatalysatorsystems kommt.

Gelöst wird diese Aufgabe durch Verbindungen der allgemeinen Formel (I) 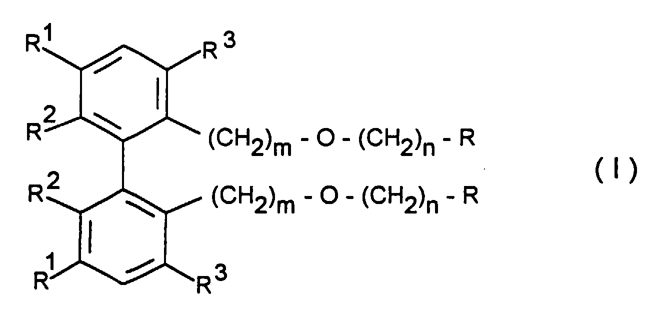
worin R¹, R² und R³ gleich oder verschieden sind und unabhängig voneinander für Wasserstoff, eine Alkyl- oder Alkoxygruppe mit 1 bis 4 Kohlenstoffatomen, eine Alkenylgruppe mit 2 bis 4 Kohlenstoffatomen stehen oder R¹ und R² unter Einschluß der jeweils mit ihnen verbundenen Kohlenstoffatome einen Ring mit 6 Kohlenstoffatomen bilden,
m und n unabhängig voneinander 0 oder 1 und (m + n) gleich 1 oder 2 ist, und
R für einen unsubstituierten oder durch eine Alkylgruppe mit 1 bis 4 Kohlenstoffatomen, eine Amino- oder Dialkylaminogruppe mit insgesamt 2 bis 8 Kohlenstoffatomen substituierten Phenyl- oder Naphthylrest steht.

Von Interesse sind Verbindungen der Formel (I) worin R¹, R² und R³ gleich oder verschieden sind und unabhängig voneinander für Wasserstoff, eine Alkyl- oder Alkoxygruppe mit 1 bis 2 Kohlenstoffatomen stehen oder R¹ und R² unter Einschluß der jeweils mit ihnen verbundenen Kohlenstoffatome einen Ring mit 6 Kohlenstoffatomen bilden.

Die aus dem Zusammenschluß von R¹ und R² resultierenden 4 Kohlenstoffatome enthaltende, den Ringschluß bewirkende Brücke ist gesättigt oder einfach oder mehrfach ungesättigt, insbesondere ungesättigt.

In den Verbindungen der Formel (I) ist insbesondere m = 1 und n = 0 oder m = 1 und n = 1.

R steht für einen unsubstituierten oder einen durch eine Alkylgrppe mit 1 bis 4 Kohlenstoffatomen substituierten Phenylrest, insbesondere für einen unsubstituierten oder durch eine Alkylgruppe mit 1 bis 2 Kohlenstoffatomen substituierten Phenylrest, bevorzugt für einen Phenylrest.
Von besonderem Interesse sind Verbindungen, die der Formel (II) oder (III) 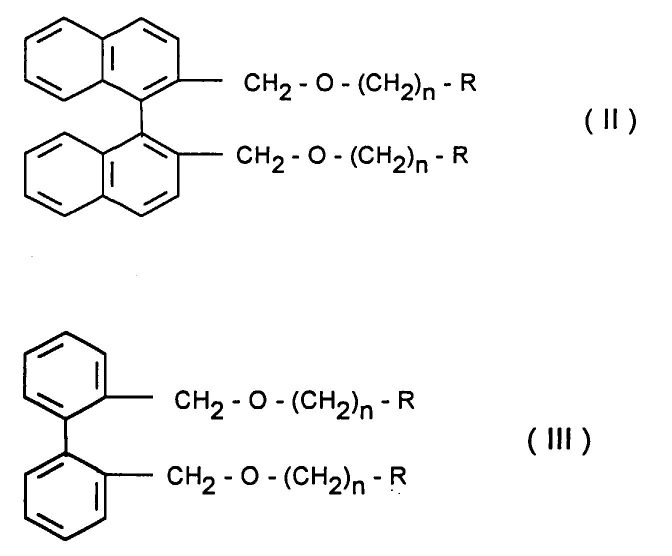 entsprechen, worin n und R die vorstehend genannte Bedeutung besitzen.

Die vorliegende Erfindung betrifft ferner ein Verfahren zur Herstellung der Verbindungen der Formel (I), respektive (II) und (III). Das Verfahren ist dadurch gekennzeichnet, daß man eine Verbindung der Formel (IV) 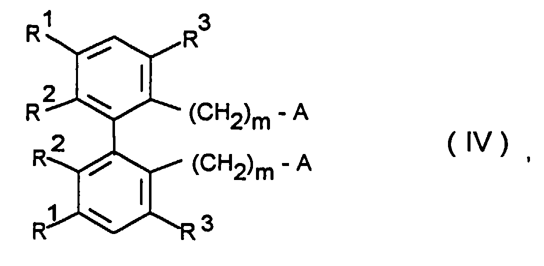 worin R¹, R², R³ und m die in Formel (I) genannte Bedeutung besitzen und A für OH oder eine Abgangsgruppe Z steht, wobei Z für Cl, Br, J oder eine R⁴OSO₂-Gruppe steht und R⁴ ein CH₃-, Phenyl-, Tolyl- oder Trialkylammonium-Rest mit 1 bis 4 Kohlenstoffatomen je Alkylrest ist, mit einer Verbindung der Formel (V)

B-(CH₂)ₙ-R (V),

worin R und n die in Formel (I) genannte Bedeutung besitzen und B für die vorstehend genannte Abgangsgruppe Z steht, falls A für OH steht, oder für OH steht, falls A die Abgangsgruppe Z ist, im Molverhältnis 1:(2 bis 2,2) in Anwesenheit einer Base und in Gegenwart eines unter den Bedingungen der Reaktion inerten organischen Lösungsmittels in Anwesenheit oder Abwesenheit eines Phasentransferkatalysators und in Anwesenheit oder Abwesenheit von Wasser bei einer Temperatur von 10 bis 100°C umsetzt.

Das Verfahren umfaßt somit zwei unterschiedliche Varianten. Bei der ersten Variante setzt man eine Verbindung der Formel (IV), worin A für OH steht, mit einer Verbindung der Formel (V), worin B für die bereits erwähnte Abgangsgruppe Z (Z = Cl, Br, J oder R⁴OSO₂) steht, um. Nach der zweiten Variante setzt man eine Verbindung der Formel (IV), worin A für die Abgangsgruppe Z steht, mit einer Verbindung der Formel (V) worin B für OH steht, um.

Die durch die beiden unterschiedlichen Verfahrensvarianten bedingte Flexibilität stellt einen zusätzlichen Vorteil des erfindungsgemäßen Verfahrens dar, da hierdurch die Zahl der für die Herstellung der einen 2,2'-Bisarylrest enthaltenden Bisether Ausgangsstoffe größer als bei einer einzigen Verfahrensvariante ist und zudem die Wahlfreiheit gegeben wird, sich nach Bedarf die am leichtest zugänglichen Ausgangsstoffe für die Synthese aussuchen zu können.

Man kann also jeweils nach Wunsch der ersten Variante des Verfahren oder der zweiten Variante des Verfahrens den Vorzug geben, wobei nicht unerwähnt bleiben sollte, daß die beiden Varianten prinzipiell als gleichwertig anzusehen sind. Es kann allerdings sein, daß man aus rein praktischen Gründen, z.B. bei leichter Zugänglichkeit eines Ausgangsstoffes, insbesondere der Verbindung der Formel (VI), einer der beiden Varianten bevorzugt anwendet.

In einer Reihe von Fällen hat es sich als nützlich erwiesen, eine Verbindung der Formel (IV), worin A für Br steht, einzusetzen. Es ist auch möglich, eine Verbindung der Formel (V), worin B für Br steht, einzusetzen.

Üblicherweise setzt man die Verbindung der Formel (IV) und die Verbindung der Formel (V) im Molverhältnis 1:(2 bis 2,2), insbesondere 1:(2 bis 2,1) ein.

Man setzt als Base ein Oxid, Hydroxid, Carbonat oder Hydrogencarbonat eines Alkalimetalls oder Erdalkalimetalls, ein tertiäres Amin oder ein Gemisch dieser Stoffe, insbesondere ein Alkalimetallhydroxid, bevorzugt Natriumhydroxid oder Kaliumhydroxid, ein.

Üblicherweise setzt man 1 bis 2 Äquivalente Base je Äquivalent der in der Verbindung der Formel (IV) oder (V) vorhandenen OH-Gruppen ein.

Als inertes organisches Lösungsmittel kann man ein aliphatisches Keton mit 3 bis 6 Kohlenstoffatomen, ein cycloaliphatisches Keton mit 5 bis 8 Kohlenstoffatomen, einen aliphatischen oder cycloaliphatischen Ether mit 4 bis 8 Kohlenstoffatomen, ein Alkylnitril mit 1 bis 6 Kohlenstoffatomen im Alkylrest, einen einfach oder mehrfach chlorierter Kohlenwasserstoff mit 1 bis 6 Kohlenstoffatomen, ein Dialkylcarbonsäureamid mit 1 bis 4 Kohlenstoffatomen je Alkylgruppe und mit 1 bis 4 Kohlenstoffatomen im Carbonsäurerest oder ein Gemisch dieser Stoffe, insbesondere Aceton, Tetrahydrofuran, Dioxan, Acetonitril, Dichlormethan, Chloroform oder ein Gemisch dieser Stoffe, bevorzugt Acetonitril oder Dichlormethan, einsetzen.

Man kann, wie bereits erwähnt, in Anwesenheit oder Abwesenheit eines Phasentransferkatalysators arbeiten. Der Gebrauch eines Phasentransferkatalysators empfiehlt sich, wenn man die Herstellung der Verbindungen der Formel (I) in einem aus zwei Phasen bestehenden System durchführt. Die Phasen können hierbei flüssig/flüssig oder flüssig/fest sein.

Als Phasentransferkatalysatoren eignen sich quartäre Ammonium- oder Phosphoniumsalze, beispielsweise Tetraalkylammonium-, Tetraphenylammonium-, Trialkylphenylammonium-, Tetraalkylphosphonium-, Tetraphenylphosphonium- oder Trialkylphenylphosphoniumsalze. Die Alkylreste können gleich oder verschieden sein und weisen in der Regel 1 bis 18, insbesondere 2 bis 16 Kohlenstoffatome auf.

Mit gutem Erfolg lassen sich quartäre Ammonium- oder Phosphoniumhalogenide, insbesondere Chloride oder Bromide einsetzen. Es lassen sich jedoch auch Kronenether, insbesondere 18-Krone-6 als Phasentransferkatalysator einsetzen.

Üblicherweise setzt man den Phasentransferkatalysator in einer Menge von 1 bis 10, insbesondere 2 bis 8 Mol.-%, bezogen auf die Verbindung der Formel (IV) ein.

Falls gewünscht, lassen sich auch Mischungen der vorstehend genannten Phasentransferkatalysatoren einsetzen.

Das Verfahren läßt sich auf einfache Weise, ohne nennenswerten technischen Aufwand zu verursachen, durchführen. Die einzelnen Ausgangsstoffe können in beliebiger Reihenfolge zugesetzt werden. Es sollte allenfalls darauf geachtet werden, daß alle Reaktanten vorliegen, bevor die vorgegebene Reaktionstemperatur eingestellt wird.

Üblicherweise vermischt man die Verbindungen der Formel (IV) und (V) mit der Base und gegebenenfalls dem Phasentransferkatalysator, setzt das unter den Bedingungen der Reaktion inerte Lösungsmittel zu und erwärmt das Gemisch unter Rühren auf die jeweils erforderliche Reaktionstemperatur.

In einer Vielzahl von Fällen genügt es, die Umsetzung bei vergleichsweise niedrigen Temperaturen, beispielsweise 15 bis 80, insbesondere 20 bis 50°C ablaufen zu lassen.
Bei Einsatz relativ reaktionsträger Ausgangsstoffe empfiehlt es sich, generell bei höheren Temperaturen, beispielsweise bis 100, insbesondere 80 bis 100°C zu arbeiten.

In einigen Fällen kann es nützlich sein, in Anwesenheit von Wasser zu arbeiten. Überlicherweise kann man auf eine Anwesenheit von Wasser verzichten und die Umsetzung in Abwesenheit von Wasser ablaufen lassen.

Ein Zusatz von Wasser ist jedoch bei der sich an die Herstellung anschließenden Aufarbeitung des Reaktionsgemisches hilfreich, da sich durch die Zugabe von Wasser unerwünschte Nebenprodukte und Verunreinigungen, beispielsweise nicht umgesetzte Base und die bei der Umsetzung gebildeten Salze, auf einfache Weise mittels Extraktion entfernen lassen.

Das erfindungsgemäße Verfahren läßt sich kontinuierlich oder diskontinuierlich, insbesondere diskontinuierlich ausführen. Man kann es bei Unterdruck, Normaldruck oder Überdruck ausüben. Üblicherweise arbeitet man bei Normaldruck.

Der Vollständigkeit halber sei an dieser Stelle darauf hingewiesen, daß ein Katalysator, der die Verbindungen der Formel (I) enthält, Gegenstand einer am gleichen Tag wie die vorliegende Patentanmeldung eingereichten deutschen Patentanmeldung (Aktenzeichen 196 25 168.0-41) ist.

Die nachfolgenden Beispiele beschreiben die Erfindung näher, ohne sie zu beschränken.

### Experimenteller Teil

### Beispiel 1

### Herstellung von 2,2'-Bis-(phenoxymethyl)-1,1'-binaphthyl

Ein Gemisch aus 11,0 g (25 mmol) 2,2'-Bis-(brommethyl)-1,1'-binaphthyl, 4,8 g (51,2 mmol) Phenol, 5,0 g (89,3 mmol) pulverisiertes Kaliumhydroxid und 1,14 g (5 mmol) Triethylbenzylammoniumchlorid wird mit 30 ml Dichlormethan versetzt und 5 Stunden lang bei 40°C gerührt.

Anschließend extrahiert man zweimal mit je 10 ml Wasser, trennt die wäßrige Phase ab, trocknet die organische Phase mit Magnesiumsulfat, filtriert und engt das Filtrat im Vakuum ein.

Man erhält 11,5 g eines farblosen Feststoffes, der aus Acetonitril umkristallisiert wird. Dabei fallen 8,8 g farblose Kristalle (entsprechend 76 % der Theorie) mit einem Schmelzpunkt von 117,6 bis 118,2°C an.

| Elementaranalyse C₃₄H₂₆O₂ (466,6) | | | |
|---|---|---|---|
| Berechnet: | C 87,5 % | H 5,6 % | O 6,9 % |
| Gefunden: | C 87,5 % | H 5,6 % | O 6,9 % |

### Beispiel 2

### Herstellung von 2,2'-Bis-(phenoxymethyl)-biphenyl

Ein Gemisch aus 25,0 g (73,5 mmol) 2,2'-Bis-(brommethyl)-biphenyl, 14,2 g (151 mmol) Phenol, 14,7 g (261 mmol) pulverisiertes Kaliumhydroxid und 3,41 g (15 mmol) Triethylbenzylammoniumchlorid wird mit 120 ml Dichlormethan versetzt und 5 Stunden lang bei 40°C gerührt.

Anschließend extrahiert man zweimal mit je 10 ml Wasser, trennt die wäßrige Phase ab, trocknet die organische Phase mit Magnesiumsulfat, filtriert und engt das Filtrat im Vakuum ein.

Man erhält 26,8 g eines farblosen Feststoffes, der aus Acetonitril umkristallisiert wird. Dabei fallen 22,8 g farblose Kristalle (entsprechend 85 % der Theorie) mit einem Schmelzpunkt von 52°C an.

| Elementaranalyse C₂₆H₂₂O₂ (366,3) | | | |
|---|---|---|---|
| Berechnet: | C 85,3 % | H 6,1 % | O 8,7 % |
| Gefunden: | C 85,2 % | H 6,1 % | O 8,7 % |

## Patentansprüche

1. Verbindungen der allgemeinen Formel (I) 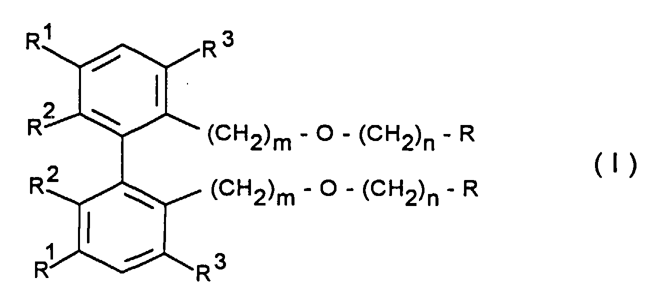 worin R¹, R² und R³ gleich oder verschieden sind und unabhängig voneinander für Wasserstoff, eine Alkyl- oder Alkoxygruppe mit 1 bis 4 Kohlenstoffatomen, eine Alkenylgruppe mit 2 bis 4 Kohlenstoffatomen stehen oder R¹ und R² unter Einschluß der jeweils mit ihnen verbundenen Kohlenstoffatome einen Ring mit 6 Kohlenstoffatomen bilden, m und n unabhängig voneinander 0 oder 1 und (m + n) gleich 1 oder 2 ist, und R für einen unsubstituierten oder durch eine Alkylgruppe mit 1 bis 4 Kohlenstoffatomen, eine Amino- oder Dialkylaminogruppe mit insgesamt 2 bis 8 Kohlenstoffatomen substituierten Phenyl- oder Naphthylrest steht.

2. Verbindungen nach Anspruch 1, dadurch gekennzeichnet, daß R¹, R² und R³ gleich oder verschieden sind und unabhängig voneinander für Wasserstoff, eine Alkyl- oder Alkoxygruppe mit 1 bis 2 Kohlenstoffatomen stehen oder R¹ und R² unter Einschluß der jeweils mit ihnen verbundenen Kohlenstoffatome einen Ring mit 6 Kohlenstoffatomen bilden.

3. Verbindungen nach Anspruch 1 oder 2, dadurch gekennzeichnet, daß m = 1 und n = 0 oder m = 1 und n = 1 ist.

4. Verbindungen nach einem oder mehreren der Ansprüche 1 bis 3, dadurch gekennzeichnet, daß R für einen unsubstituierten oder einen durch eine Alkylgruppe mit 1 bis 4 Kohlenstoffatomen substituierten Phenyl- oder Naphthylrest steht.

5. Verbindungen nach einem oder mehreren der Ansprüche 1 bis 4, dadurch gekennzeichnet, daß R für einen unsubstituierten oder einen durch eine Alkylgruppe mit 1 bis 4 Kohlenstoffatomen substituierten Phenylrest steht.

6. Verbindungen nach einem oder mehreren der Ansprüche 1 bis 4, dadurch gekennzeichnet, daß R für einen Phenylrest steht.

7. Verbindungen nach einem oder mehreren der Ansprüche 1 bis 6, dadurch gekennzeichnet, daß sie der Formel (II) oder (III) 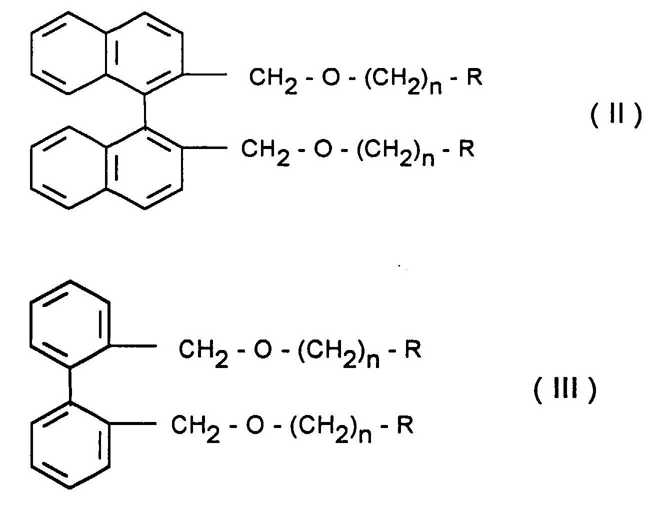 entsprechen, worin n und R die vorstehend genannte Bedeutung besitzen.

8. Verfahren zur Herstellung der Verbindungen der Formel (I) nach einem oder mehreren der Ansprüche 1 bis 7, dadurch gekennzeichnet, daß man eine Verbindung der Formel (IV) 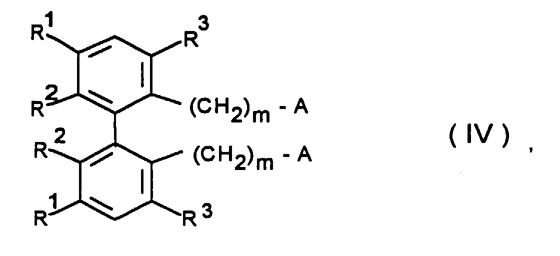 worin R¹, R², R³ und m die in Formel (I) genannte Bedeutung besitzen und A für OH oder eine Abgangsgruppe Z steht, wobei Z für Cl, Br, J oder eine R⁴OSO₂-Gruppe steht und R⁴ ein CH₃-, Phenyl-, Tolyl- oder Trialkylammonium-Rest mit 1 bis 4 Kohlenstoffatomen je Alkylrest ist, mit einer Verbindung der Formel (V)
B-(CH₂)ₙ-R (V)
worin R und n die in Formel (I) genannte Bedeutung besitzen und B für die vorstehend genannte Abgangsgruppe Z steht, falls A für OH steht, oder für OH steht, falls A die Abgangsgruppe Z ist, im Molverhältnisl 1:(2 bis 2,2) in Anwesenheit einer Base und in Gegenwart eines unter den Bedingungen der Reaktion inerten organischen Lösungsmittels in Anwesenheit oder Abwesenheit eines Phasentransferkatalysators und in Anwesenheit oder Abwesenheit von Wasser bei einer Temperatur von 10 bis 100°C umsetzt.

9. Verfahren nach Anspruch 8, dadurch gekennzeichnet, daß man eine Verbindung der Formel (IV), worin A für Br steht, umsetzt.

10. Verfahren nach Anspruch 8, dadurch gekennzeichnet, daß man eine Verbindung der Formel (V), worin B für Br steht, einsetzt.

11. Verfahren nach einem oder mehreren der Ansprüche 8 bis 10, dadurch gekennzeichnet, daß man die Verbindung der Formel (IV) und die Verbindung der Formel (V) im Molverhältnis 1: (2 bis 2,2) einsetzt.

12. Verfahren nach einem oder mehreren der Ansprüche 8 bis 11, dadurch gekennzeichnet, daß man als Base ein Oxid, Hydroxid, Carbonat oder Hydrogencarbonat eines Alkalimetalls oder Erdalkalimetalls, oder ein tertiäres Amin oder ein Gemisch dieser Stoffe einsetzt.

13. Verfahren nach einem oder mehreren der Ansprüche 8 bis 12, dadurch gekennzeichnet, daß man als Base ein Alkalimetallhydroxid einsetzt.

14. Verfahren nach einem oder mehreren der Ansprüche 8 bis 13, dadurch gekennzeichnet, daß man als Base Natriumhydroxid oder Kaliumhydroxid einsetzt.

15. Verfahren nach einem oder mehreren der Ansprüche 8 bis 14, dadurch gekennzeichnet, daß man 1 bis 2 Äquivalente Base je Äquivalent der in der Verbindung der Formel (IV) oder (V) vorhandenen OH-Gruppen einsetzt.

16. Verfahren nach einem oder mehreren der Ansprüche 8 bis 15, dadurch gekennzeichnet, daß man als inertes organisches Lösungsmittel ein aliphatisches Keton mit 3 bis 6 Kohlenstoffatomen, ein cycloaliphatisches Keton mit 5 bis 8 Kohlenstoffatomen, einen aliphatischen oder cycloaliphatischen Ether mit 4 bis 8 Kohlenstoffatomen, ein Alkylnitril mit 1 bis 6 Kohlenstoffatomen im Alkylrest, einen einfach oder mehrfach chlorierten Kohlenwasserstoff mit 1 bis 6 Kohlenstoffatomen, ein Dialkylcarbonsäureamid mit 1 bis 4 Kohlenstoffatomen je Alkylgruppe und mit 1 bis 4 Kohlenstoffatomen im Carbonsäurerest oder ein Gemisch dieser Stoffe einsetzt.

17. Verfahren nach einem oder mehreren der Ansprüche 8 bis 16, dadurch gekennzeichnet, daß man als inertes organisches Lösungsmittel Aceton, Tetrahydrofuran, Dioxan, Acetonitril, Dichlormethan, Chloroform oder ein Gemisch dieser Stoffe einsetzt.

18. Verfahren nach einem oder mehreren der Ansprüche 8 bis 17, dadurch gekennzeichnet, daß man Acetonitril oder Dichlormethan als inertes organisches Lösungsmittel einsetzt.

19. Verfahren nach einem oder mehreren der Ansprüche 8 bis 18, dadurch gekennzeichnet, daß man als Phasentransferkatalysator ein quartäres Ammonium- oder Phosphoniumsalz oder einen Kronenether einsetzt.
